# EUROPÄISCHE PATENTSCHRIFT

(11) **EP 0 594 028 B1**
(45) Veröffentlichungstag und Bekanntmachung des Hinweises auf die Patenterteilung: **17.05.1995**
(21) Anmeldenummer: 93116436.2
(22) Anmeldetag: 11.10.1993
(51) Int. Cl.: C07C 11/02, C07C 13/18, C07C 13/10, C07C 1/26, C07C 1/28

(54) **Verfahren zur Herstellung von Alkenen und von Cyclopentan und Cyclohexan**
Process for the preparation of alkenes and of cyclopentane and cyclohexane
Procédé pour la préparation d'alcènes et du cyclopentane et du cyclohexane

(30) Priorität: 20.10.1992 DE 4235326
(43) Veröffentlichungstag der Anmeldung: 27.04.1994
(73) Patentinhaber: BASF Aktiengesellschaft, 67063 Ludwigshafen (DE)
(72) Erfinder: Wettling, Thomas, Dr., D-6703 Limburgerhof (DE); Henkelmann, Jochem, Dr., D-6800 Mannheim 1 (DE)

(56) Entgegenhaltungen:
- DD-A- 235 630
- FR-A- 2 515 002
- US-A- 2 553 797
- US-A- 3 450 782

## Beschreibung

Die vorliegende Erfindung betrifft ein neues Verfahren zur Herstellung von Alkenen sowie von Cyclopentan und Cyclohexan aus den entsprechenden vicinalen Dichlor- und Dibromalkanen bzw. aus α,ω-Dichlor- oder Dibromalkanen bzw. aus α,ω-Dichlor- oder Dibromhexan.

Chlorierte und bromierte Kohlenwasserstoffe werden im allgemeinen durch Verbrennung entsorgt. Wegen der dabei notwendigen Abgasreinigung sind solche Verbrennungsprozesse verfahrenstechnisch aufwendig.

Die US-A 4 435 379 lehrt ein Verfahren zur Umsetzung von chlorierten Kohlenwasserstoffen mit verschiedenen Metalloxiden zu CO, CO₂ und den entsprechenden Metallchloriden. Die dazu benötigten Temperaturen liegen bei über 700°C und erfordern somit einen großen Energieaufwand.

Die Umsetzung von Monochloralkanen oder - aromaten mit Silicium hat als Rochow-Verfahren insbesondere zur Herstellung von Methyl- und Phenylchlorsilanen technische Bedeutung erlangt (US-A 2 380 995).

Die Reaktion von Silicium mit α,ω-Dihalogenalkanen mit 1 bis 4 Kohlenstoffatomen führt nach der Lehre der US-A 2 381 000 zu α,ω-Bissilylverbindungen.

Es ist bekannt, vicinale aliphatische Dihalogenide mit Metallen wie Natrium, Lithium, Magnesium und vor allem Zink zu dehalogenieren. Dabei fallen die entsprechenden Metallhalogenide als Nebenprodukte an (Houben-Weyl, Methoden der Org. Chem., Bd. V/1b, Thieme Verlag 1972, S. 182-190).

Es bestand die Aufgabe, ein Verfahren bereitzustellen, das die Herstellung von Alkenen sowie von Cyclopentan und Cyclohexan durch Enthalogenierung der entsprechenden Dichlor- bzw. Dibromverbindungen in einfacher Weise erlaubt. Bei diesem Verfahren sollten neben den Kohlenwasserstoffen Wertprodukte entstehen.

Demgemäß wurde ein Verfahren zur Herstellung von Alkenen sowie von Cyclopentan und Cyclohexan aus den oben genannten Ausgangsverbindungen gefunden, das dadurch gekennzeichnet ist, daß man diese Ausgangsverbindungen mit Silicium umsetzt.

Als Ausgangsstoffe für das erfindungsgemäße Verfahren kommen Dichlor- und Dibromverbindungen in Betracht, von denen die Dichlorverbindungen bevorzugt werden.

Bei den Ausgangsverbindungen handelt es sich in erster Linie um Nebenprodukte, die bei der technischen Chlorierung oder Bromierung von Kohlenwasserstoffen wie insbesondere Propan, Butan, Pentan und Hexan anfallen.

Besondere Bedeutung hat das Verfahren für die Dehalogenierung von vicinalen Dichlor- und Dibromalkanen wie 2,3-Dichlorbutan und insbesondere 1,2-Dichlorpropan.

Weiterhin handelt es sich bei den Ausgangsverbindungen um α,ω-Dichlor- und Dibrompentan sowie Dichlor- und Dibromhexan, von denen 1,6-Dichlorhexan bevorzugt ist. Die Verbindungen können noch weitere inerte Substituenten wie Alkylgruppen tragen.

Als Dehalogenierungsmittel wird Silicium oder Silicium in Form seiner Legierungen verwendet. Bevorzugt wird die Verwendung von relativ reinem Silicium mit beispielsweise 98 bis mehr als 99,9 Gew.-% Siliciumgehalt. Es kann auch technisches Rohsilicium mit einem Siliciumgehalt von etwa 90 bis 98 Gew.-% verwendet werden. Weiterhin eignen sich Siliciumlegierungen wie Ferrosilicium mit einem Siliciumgehalt von vorzugsweise mehr als 50 Gew.-% Silicium.

Bei Verwendung von nicht ganz reinem Silicium oder von Siliciumlegierungen erhält man zwar Nebenprodukte, jedoch in relativ geringer Menge, so daß eine Aufarbeitung des Reaktionsgemisches nicht wesentlich erschwert wird.

Da es sich bei der Umsetzung um eine heterogene Reaktion handelt, ist es um so günstiger, je feiner verteilt das Silicium vorliegt. Es empfiehlt sich daher, Silicium oder Siliciumlegierungen in Form von Pulvern oder Grieß mit einer mittleren Teilchengröße von etwa 0,05 bis 0,5 mm zu wählen.

Man kann das Silicium in stöchiometrischen Mengen einsetzen, d.h. 1 Äquivalent Silicium für 4 Äquivalente Halogen, jedoch empfiehlt sich zur Beschleunigung der Reaktion ein Überschuß an Silicium.

Nicht umgesetztes Silicium kann isoliert und in folgenden Reaktionsansätzen eingesetzt werden.

Als besonders günstig hat sich erwiesen, dem Silicium Kupfer(I)chlorid zuzusetzen. Die dafür geeigneten Mengen liegen in der Regel bei 0,1 bis 20 Gew.-%, bezogen auf das eingesetzte Silicium. Zur Aktivierung dieses Zusatzes hat sich Erhitzen auf 250 bis 300°C vor Beginn der Reaktion bewährt.

Weiterhin können als Zusätze andere Metalle oder Metallverbindungen wie Zinkoxid, Manganoxid oder aber Hauptgruppenelemente wie Schwefel oder Hauptgruppenelementverbindungen wie Phosphorpentoxid verwendet werden. Die Mengen liegen im allgemeinen bei 0,001 bis 1 Gew.-%, bezogen auf Silicium.

Das Verfahren läßt sich in unterschiedlichen Varianten ausführen.

Für halogenierte Kohlenwasserstoffe, die bei der Reaktionstemperatur und Normaldruck gasförmig sind, ist es technisch besonders vorteilhaft, sie gasförmig durch ein Wirbelbett aus Siliciumpulver zu leiten. Die Produkte können dann kondensiert und in bekannter Weise getrennt werden.

Halogenierte Kohlenwasserstoffe, die bei der Reaktionstemperatur flüssig oder fest vorliegen, können einfach unter Abdestillieren flüchtiger Reaktionsprodukte mit Silicium erhitzt werden.

Die Reaktionstemperatur liegt üblicherweise bei 100 bis 600°C, vorzugsweise bei 150° bis 350° und besonders bevorzugt bei 180° bis 320°C.

In der Regel wird die Reaktion bei Normaldruck ausgeführt, sie kann aber auch bei Unterdruck oder bei erhöhten Drücken durchgeführt werden. Das Verfahren kann sowohl kontinuierlich wie auch diskontinuierlich ausgeübt werden.

Die Reaktion wird vorzugsweise in Abwesenheit von Sauerstoff ausgeführt, da es sonst zur Oxidation der organischen Verbindungen und bei hoher Temperatur auch zu der des Siliciums kommen kann. Man kann die Reaktion unter Schutzgas wie einem Edelgas oder Stickstoff vornehmen.

Als Reaktionsprodukte bilden sich chlor- oder bromhaltige Silane, in den bevorzugten Ausführungsformen überwiegend Siliciumtetrachlorid.

Das erfindungsgemäße Verfahren erlaubt die Überführung vicinaler Dichlor- und Dibromalkane in die entsprechenden Olefine, die direkt für weitere Synthesen genutzt werden können. Weiter erlaubt es die Bildung von Cyclopentan und Cyclohexan aus den entsprechenden offenkettigen α,ω-Dihalogenalkanen, welche z.B. als Lösungsmittel Verwendung finden. Die im Verfahren gebildeten Brom- oder Chlorsilane finden eine breite technische Anwendung.

### Beispiele

### Beispiel 1

### Herstellung von Propen aus 1,2-Dichlorpropan

140 g (5 mol) Siliciumpulver (mittlere Teilchengröße 0,2 mm, Reinheit 99 %) und 14 g (10 Gew.-%) Kupfer(I)chlorid wurden 1 h auf 265°C erhitzt. Bei 230°C wurden über 3 h 115 g (1 mol) 1,2-Dichlorpropan im Stickstoffstrom in ein Wirbelbett aus dem vorbehandelten Silicium/CuCl eingetragen. Die gasförmigen Reaktionsprodukte wurden kondensiert und destilliert. Es wurden 20,8 g (0,5 mol) Propen (Selektivität 87 %), 45 g (0,27 mol) Siliciumtetrachlorid (Selektivität 93 %) und 49,5 g nicht umgesetztes 1,2-Dichlorpropan isoliert. Der Umsatz betrug 57 %.

### Beispiel 2

### Herstellung von Butenen aus 2,3-Dichlorbutan

In Analogie zu Beispiel 1 wurden bei 250°C in 2 h 55 g (0,44 mol) 2,3-Dichlorbutan umgesetzt. Die Kondensationsprodukte wurden gaschromatographisch analysiert. Man erhielt 12 % Siliciumtetrachlorid, 27 % Butene, 23 % Monochlorbutene und 16 % 2,3-Dichlorbutan (alle %-Angaben sind GC-Flächen-%, weiterhin fielen nicht identifizierte Produkte an).

### Beispiel 3

### Herstellung von Cyclohexan aus 1,6-Dichlorhexan

In Analogie zu Beispiel 1 wurden 54 g (0,35 mol) 1,6-Dichlorhexan bei 280°C in 2 h umgesetzt. Die Kondensationsprodukte wurden gaschromatographisch untersucht. Man erhielt 32 % Trichlorsilan, 2 % Siliciumtetrachlorid, 23 % 6-Chlorhex-1-en, 8 % Cyclohexan und 18 % 1,6-Dichlorhexan (alle %-Angaben sind GC-Flächen-%, weiterhin fielen nicht identifizierte Produkte an).

## Patentansprüche

1. Verfahren zur Herstellung von Alkenen sowie von Cyclopentan und Cyclohexan aus den entsprechenden vicinalen Dichlor- oder Dibromalkanen bzw. aus α,ω-Dichlor- oder Dibrompentan und α,ω-Dichlor- oder Dibromhexan, dadurch gekennzeichnet, daß man diese Ausgangsverbindungen mit Silicium umsetzt.

2. Verfahren nach Anspruch 1, dadurch gekennzeichnet, daß man die Umsetzung in Gegenwart von Kupfer(I)chlorid vornimmt.

3. Verfahren nach Anspruch 1 oder 2, dadurch gekennzeichnet, daß man 1,2-Dichlorpropan umsetzt.

## Claims

1. A process for the preparation of alkenes and of cyclopentane and cyclohexane from the corresponding vicinal dichloro- or dibromoalkanes and from α,ω-dichloro- or dibromopentane and α,ω-dichloro- or dibromohexane, wherein these starting compounds are reacted with silicon.

2. A process as claimed in claim 1, wherein the reaction is carried out in the presence of copper(I) chloride.

3. A process as claimed in claim 1 or 2, wherein 1,2-dichloropropane is reacted.

## Revendications

1. Procédé de préparation d'alcènes, ainsi que de cyclopentane et de cyclohexane à partir des dichloro- ou dibromoalcanes vicinaux correspondants ou d'α,ω-dichloro- ou -dibromopentane et d'α,ω-dichloro- ou -dibromohexane, caractérisé en ce qu'on fait réagir ces composés de départ avec du silicium.

2. Procédé selon la revendication 1, caractérisé en ce qu'on procède à la réaction en présence de chlorure de cuivre (I).

3. Procédé selon la revendication 1 ou 2, caractérisé en ce qu'on fait réagir du 1,2-dichloropropane.
